(19) Europäisches Patentamt
European Patent Office
Office européen des brevets

(11) **EP 2 324 850 A1**

(12) **EUROPEAN PATENT APPLICATION**

(43) Date of publication:
**25.05.2011 Bulletin 2011/21**

(51) Int Cl.:
*A61K 39/35* (2006.01)  *A61K 9/00* (2006.01)

(21) Application number: **11155971.2**

(22) Date of filing: **04.10.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **04.10.2005 DK 200501392**
**04.10.2005 US 722948 P**

(62) Document number(s) of the earlier application(s) in accordance with Art. 76 EPC:
**06791437.4 / 1 933 867**

(71) Applicant: **Alk-Abelló A/S**
**2970 Hørsholm (DK)**

(72) Inventors:
 • **Hejl, Charlotte**
 **1850, Frederiksberg C (DK)**

 • **Mærkedahl, Lise Lund**
 **3480, Fredensborg (DK)**
 • **Ipsen, Hans-Henrik**
 **3400, Hillerød (DK)**
 • **Sønderkær, Susanne**
 **3650, Ølstykk (DK)**
 • **Lundegaard, Annette Rømmelmayer**
 **2860, Søborg (DK)**
 • **Seppälä, Ulla**
 **254 51, Helsingborg (SE)**

(74) Representative: **Inspicos A/S**
**Kogle Allé 2**
**P.O. Box 45**
**2970 Hørsholm (DK)**

Remarks:
This application was filed on 25-02-2011 as a divisional application to the application mentioned under INID code 62.

(54) **Solid vaccine formulation**

(57) The invention relates to a solid vaccine formulation adapted for mucosal administration comprising at least one antigen as active substance, wherein the formulation comprises a lyophilisate of a suspension comprising an oxygen-containing metal salt, the antigen(s) and one or more excipients selected from (i) saccharides, (ii) sugar alcohols, and (iii) amino acids or pharmaceutically acceptable salts thereof.

EP 2 324 850 A1

**Description**

TECHNICAL FIELD

The present invention relates to a solid vaccine formulation adapted for mucosal administration and comprising at least one antigen as active substance, wherein the formulation comprises a lyophilisate of a suspension comprising an oxygen-containing metal salt, the antigen(s) and at least one excipient selected from (i) saccharides, (ii) sugar alcohols and (iii) amino acids or pharmaceutically acceptable salts thereof. The invention also relates to the use of a lyophilisate as above for the preparation of a solid vaccine formulation for mucosal administration and the use of such formulations for vaccination or treatment of allergy or alleviating symptoms of allergy in a subject.

BACKGROUND OF THE INVENTION

Trehalose is a non-reducing disaccharide consisting of two glucose monomers. It is present in a number of organisms, including bacteria, fungi and vertebrates, as well as a few plants. Trehalose is known to stabilise proteins.

US-A-4 578 270 discloses a method of preparing a lyophilised vaccine, wherein a mixture of an antigen absorbed on an insoluble carrier, such as aluminium hydroxide and aluminium phosphate gel, and a protective agent is subjected to lyophilisation. The protective agent may be e.g. proteins, polypeptides, polysaccharides and other synthetic protective colloids. Specifically, dextran having a molecular weight of 40,000 and a combination of dextran and polysaccharides are used. The protective agent is used in an amount of from 0.5 to 10 % of the solution of the antigen. Upon application the lyophilized vaccine is reconstituted with a buffer.

US-A-5 902 565 discloses a method of preparing a vaccine preparation in the form of a dry microspherical particles, which can subsequently be incorporated into a liquid formulation or into a solid pellet or implant. The gel-forming nature of aluminium gels is completely retained. One aspect of US-A-5 902 565 is directed to a method of producing an immediate-release vaccine comprising forming an aqueous suspension of immunogen adsorbed to an aluminium salt adjuvant and spray-drying the suspension. Optionally, the suspension contains a protein stabiliser, such as sugars and sugar derivatives, e.g. trehalose, dextrose, and glucosamine.

EP-B1-0 130 619 discloses a method of preparing a lyophilised vaccine preparation of inactivated, purified hepatitis B virus surface antigen comprising adding an aluminium gel and a stabiliser to the antigen and lyophilising the mixture. The stabiliser may be amino acids, colloidal substances and polysaccharides, such as monosaccharides, disaccharides, e.g. lactose, maltose and saccharose, and sugar alcohols.

Gribbon et al. (Dev Biol Stand. Basel, Karger, 1996, vol 87, pp 193-199) discloses a study of the stabilisation of vaccines using trehalose as stabiliser. A commercial preparation of diphteria and tetanus antigen vaccine adjuvanted with alum was mixed with trehalose, and the mixture was subjected to freeze-drying to obtain a dry powder. The powder was stored at 45 °C for up to 35 weeks, and the activity of reconstituted tetanus toxoid was measured. After 35 weeks, the recovered activity was 94 % compared to corresponding fresh wet controls. Furthermore, the stabilising and protective effect of trehalose was compared to glucose and sucrose, and it was found that trehalose was more effective.

Maa et al. (Journal of Pharmaceutical Sciences, Vol. 92, No.2, February 2003) presents a study on stabilisation of alum-adjuvanted vaccine dry powder formulations comprising Hepatitis B surface antigen or Diptheria and Tetanus toxoids. Various drying techniches were tested, and it was found that Spray Freeze Drying was superior to Freeze Drying, Spray Drying and Air Drying in preventing alum gel coagulation and in retaining immunogenicity. Also, the effect of various stabilisers on preventing alum gel coagulation was tested, and it was found that the effect was only minor.

Maa et al. (Pharmaceutical Research, Vol. 20, No. 7, July 2003) discloses a Spray Freeze Drying process for producing alum-adsorbed Hepatitis B surface antigen vaccine powders, wherein a combination of trehalose, mannitol and dextran, preferably in a ratio of 3/3/4, is used as stabiliser. The article describes epidermal powder immunidsation (EPI) and do no suggest mucosal administration.

WO 02/101412 discloses a process for the preparation of a powder, wherein an aqueous solution or suspension is spray-freeze-dried. The aqueous solution has a solids content of at least 20 % by weight and contains one or more excipients selected among 1) an aluminium salt or calcium salt adjuvant, 2) an amorphous excipient, e.g. trehalose, 3) a crystalline excipient, e.g. mannitol, 4) a polymer, e.g. dextran. A number of specific alum-adjuvanted antigen compositions are mentioned including combinations of (a) trehalose, mannitol and dextran, and (b) trehalose, glycine, and dextran. The powder is described as useful for administration via a needleless syringe device.

WO 01/93829 relates to a gel-forming free-flowing powder suitable for use as a vaccine which is prepared by spray-drying or spray freeze-drying an aqueous suspension that contains an antigen adsorbed to an aluminum salt or calcium salt adjuvant, a saccharide, an amino acid or a salt thereof, and a colloidal substance. The powder is said to be useful for transdermal delivery of vaccines using a needleless syringe.

WO 95/33488 discloses a method of reducing or preventing aggregation during dehydration and rehydration of a substance, such as aluminium hydroxide and aluminium phosphate. The document presents an experimental study of the effect of trehalose on the aggregation of aluminium phosphate, wherein a suspension of aluminium phosphate in the presence and absence of 15 % trehalose is subjected to i.a. freeze-drying. It was found that the trehalose in this concentration prevented aggregation upon rehydration.

Vaccine formulations

Conventional specific allergy vaccination is carried out using multiple subcutaneous immunizations applied over an extended time period. The course is divided in two phases, the up dosing and the maintenance phase. In the up dosing phase increasing doses are applied, typically over a 16-week period, starting with minute doses. When the recommended maintenance dose is reached, this dose is applied for the maintenance phase, typically with injections every six weeks. Following each injection the patient must remain under medical attendance for 30 minutes due to the risk of anaphylactic side reactions, which in principle although extremely rare could be life-threatening. In addition, the clinic should be equipped to support emergency treatment. There is no doubt that a vaccine based on a different route of administration would eliminate or reduce the risk for allergic side reactions inherent in the current subcutaneous based vaccine as well as would facilitate a more widespread use, possibly even enabling self vaccination at home.

In conventional allergy vaccines for subcutaneous administration the allergen is typically present in a suspension of aluminium hydroxide gel serving as an adjuvant. The mechanisms behind the function of aluminium hydroxide as an adjuvant are not fully understood, but it is believed that the adjuvant effect of aluminium hydroxide is due to a depot effect hence facilitating a prolonged release of allergen to the body.

Vaccine formulations based on aluminium hydroxide have the disadvantage that they require refrigeration during storage and transport. Also, freezing and lyophilisation reduce the potency and physical properties, such as sedimentation velocity and fluidity, of the vaccine formulation.

WO 04/047794 discloses a fast-dissolving, non-compressed solid allergen vaccine dosage form suitable for oromucosal administration of an allergen comprising an allergen as active agent and a matrix composed of a matrix forming agent, such as gelatine or starch.

WO 00/45847 suggests a vaccine for delivery of an immunogenic substance via a mucosal membrane comprising at least one immunogenic substance and a mucosal delivery system comprising an oxygen-containing metal salt. In the examples of this application the vaccines are administered by oral gavage (directly to the stomach), or intraperitoneally as solutions. Thus, there are no examples describing a solid formulation for mucosal administration, such as sublingual administration.

EP 1 516 615 relates to a solid dose mucoadhesive wafer or film formulation suitable for transmucosal delivery where the wafer of film is formed from a stabilising polyol and a therapeutic agent dissolved or dispersed therein. The polyol may for example be trehalose and the therapeutic agent may be an antigen and adjuvants. This application does not suggest a solid vaccine formulation which comprises a lyophilisate of an aqueous suspension of an oxygen-containing metal salt, an antigen and one or more excipients, and in particular not a vaccine formulation comprising a lyophilisate of an aqueous suspension comprising allergen, aluminium hydroxide and one or more excipients.

US 5,591,433 relates to oral formulations comprising allergen microencapsulated in an enteric coating.

The object of the present invention is to provide an improved solid vaccine formulation for mucosal administration.

SUMMARY OF THE INVENTION

The present invention relates to a solid vaccine formulation comprising a lyophilisate of a suspension comprising an oxygen-containing metal salt, at least one antigen, and at least one excipient selected from (i) saccharides, (ii) sugar alcohols and (iii) amino acids or pharmaceutically acceptable salts thereof.

The invention also relates to the use of a lyophilisate of a suspension comprising an oxygen-containing metal salt, at least one antigen, and at least one excipient selected from (i) saccharides, (ii) sugar alcohols and (iii) amino acids or pharmaceutically acceptable salts thereof, for the preparation of a solid vaccine formulation for mucosal administration.

In a further aspect, the invention relates to a method for vaccination of a subject comprising mucosal administration of an effective amount of a solid formulation as above.

In particular the invention relates to a method for treatment of allergy or alleviating symptoms of allergy comprising mucosal administration of an effective amount of a solid formulation as above comprising at least one allergen.

The present invention is based on the recognition that it would be desirable to include an adjuvant in a solid vaccine formulation for mucosal administration, and also that it would be desirable if the adjuvant has mucosa-adhesive properties. Furthermore, it is realised that oxygen-containing metal salts, such as aluminium hydroxide, has both adjuvant and mucosa-adhesive properties thus making such substances ideal for use in solid vaccine formulation for mucosal administration.

The invention is based on the finding that trehalose, sucrose and certain other excipients has highly exceptional properties with respect to protecting aluminium hydroxide and corresponding oxygen-containing metal salts against the detrimental effects of freeze-drying and that hence aluminium hydroxide-containing solid matter obtained by freeze-drying of a suspension of aluminium hydroxide and trehalose, sucrose and certain other excipients is capable of being readily re-hydrated to form a aluminium gel with properties, which to a surprising level are unaffected by the freeze-drying process.

The present invention is further based on the recognition that the above-mentioned surprising functionalities of trehalose, sucrose and certain other excipients may be utilised to prepare a solid vaccine formulation, such as a fast-dissolving tablet, designed to re-hydrate when contacted with a mucosa, which contains an oxygen-containing metal salt, such as aluminium hydroxide, adjuvant.

Thus, the present invention has provided a possibility of preparing a solid vaccine formulation for mucosal administration containing an oxygen-containing metal salt, such as aluminium hydroxide, adjuvant.

SHORT DESCRIPTION OF THE FIGURES

Fig. 1 shows the degree of inhibition of the binding of Der p-specific IgE to biotinylated Der p by various inhibitor formulations

Fig. 2 shows the degree of inhibition of the binding of Der p-specific IgE to biotinylated Der p by various inhibitor formulations.

Fig. 3 shows a comparison of potency estimates for three test pairs of formulations.

Fig. 4 shows the level of Phl p specific IgG in mice immunised three times with the different Phl p formulations.

Fig. 5 shows the level of Der p 1 specific IgG in mice immunised two times (top panel) and five times (top panel) with different Der p formulations.

Fig. 6 illustrates the temporal development of the Der p 1 specific IgG during the course of the immunisation program.

DETAILED DESCRIPTION OF THE INVENTION

The present invention provides a solid vaccine formulation comprising a lyophilisate of a suspension comprising an oxygen-containing metal salt, at least one antigen and at least one excipient selected from the group consisting of (i) saccharides, (ii) sugar alcohols and (iii) amino acids or pharmaceutically acceptable salts thereof.

The invention also relates to the use of a lyophilisate of a suspension comprising an oxygen-containing metal salt, at least one antigen and at least one excipient selected from the group consisting of (i) saccharides, (ii) sugar alcohols and (iii) amino acids or pharmaceutically acceptable salts thereof, for the preparation of a solid vaccine formulation.

The suspension is suitably an aqueous suspension.

In one embodiment of the invention, the suspension comprises one excipient selected from saccharides.

In another embodiment of the invention, the suspension comprises one excipient selected from sugar alcohols.

In still another embodiment of the invention, the suspension comprises one excipient selected from amino acids and pharmaceutically acceptable salts thereof.

In a further embodiment of the invention, the suspension comprises one excipient selected from saccharides, and one excipient selected from sugar alcohols.

In a further embodiment of the invention, the suspension comprises one excipient selected from saccharides and one excipient selected from amino acids and pharmaceutically acceptable salts thereof.

In a further embodiment of the invention, the suspension comprises one excipient selected from the sugar alcohols and one excipient selected from amino acids and pharmaceutically acceptable salts thereof.

In one embodiment of the invention as above, the suspension comprises one excipient selected from saccharides, one excipient selected from the sugar alcohols and one excipient selected from amino acids and pharmaceutically acceptable salts thereof.

As used herein saccharides means monosaccharides, disaccharides and oligosaccharides.

Suitably the saccharide is selected from dextrose, sucrose, lactose, trehalose, cellobiose, raffinose, isomaltose and cyclodextrins.

Preferably, the saccharide is selected from the group consisting of trehalose and sucrose.

As used herein a sugar alcohol means a hydrogenated form of a saccharide (suitably a monosaccharide) where a carbonyl group of the saccharide has been reduced to a primary or secondary hydroxyl group.

Suitably, the sugar alcohol is selected from the group consisting of mannitol, sorbitol, xylitol, glycerol, erythtritol, arabitol and allitol, and preferably the sugar alcohol is mannitol.

Suitably the amino acid is selected from glycine, alanine, glutamine, arginine, lysine, proline, serine and histidine and pharmaceutically acceptable salts thereof. Preferably the amino acid is glycine.

The salts of the amino acids may be alkali or alkaline earth metal salts such as sodium, potassium or magnesium salts or salts with other amino acids such as glutamate or aspartate salts.

In a further embodiment, the suspension comprises an additional excipient which is a polymer. The polymer may be selected from dextran, maltodextran, starch, cellulose, gelatine, agarose, polyvinylpyrrolidone (PVP), polyvinyl-lalcohol (PVA), and polyethylene glycols (PEG). Preferably the polymer is dextran.

Thus, in one embodiment of the invention, the suspension comprises one excipient selected from saccharides, such as trehalose and sucrose, one excipient selected from the sugar alcohols, such as mannitol, and one excipient which is a polymer, such as dextran.

In a further embodiment of the invention, the suspension comprises one excipient selected from saccharides, such as trehalose or sucrose, and one excipient selected from amino acids and pharmaceutically acceptable salts thereof, such as glycine, and one excipient which is a polymer, such as dextran.

In a further embodiment of the invention, the suspension comprises one excipient selected from the sugar alcohols, such as mannitol, and one excipient selected from amino acids and pharmaceutically acceptable salts thereof, such as glycine, and one excipient which is a polymer, such as dextran.

In another embodiment of the invention, the suspension comprises one excipient selected from saccharides, such as trehalose or sucrose, and one excipient which is a polymer, such as dextran.

In another embodiment of the invention, the suspension comprises one excipient selected from sugar alcohols, such as mannitol, and one excipient which is a polymer, such as dextran.

In a further embodiment of the invention, the suspension comprises one excipient selected from amino acids and pharmaceutically acceptable salts thereof, such as glycin, and one excipient which is a polymer, such as dextran.

Various grades of dextran are available and a suitable dextran has a molecular weight greater than 10, 000 such as a mean molecular weight in the range from 10,000 to 200,000. Preferably the dextran have molecular weight of 40.000, 60.000 or 70.000 dalton.

According to one embodiment of the invention, the suspension comprises a mixture of sucrose and mannitol.

In another embodiment of the invention, the suspension comprises a mixture of sucrose, glycine and dextran.

In another embodiment of the invention, the suspension comprises a mixture of trehalose and mannitol.

In another embodiment of the invention, the suspension comprises a mixture of sucrose, mannitol and dextran.

In another embodiment of the invention, the suspension comprises a mixture of trehalose, glycine, and dextran.

In another embodiment of the invention, the suspension comprises a mixture of trehalose, mannitol and dextran.

In another embodiment of the invention, the suspension comprises a mixture of trehalose and glycine.

In another embodiment of the invention, the suspension comprises a mixture of sucrose and glycine.

In another embodiment of the invention, the suspension comprises a mixture of glycine and mannitol.

A preferred combination of excipients for use in the present invention is the combination of a saccharide, a sugar alcohol or an amino acid or a pharmaceutically acceptable salt thereof, and a polymer.

When the suspension comprises sucrose and mannitol the weight ratio of sucrose to mannitol is suitably from 1:4 to 4:1, or from 1:2 to 2:1, e.g. 1:4, 3:2, 2:3, 4:1, 2:1, 1:2 or 1:1, and preferably the ratio is 1:1.

When the suspension comprises sucrose, glycine and dextran, the weight ratio of sucrose to glycine to dextran is suitably selected from 5:4:1, 4:5:1, 3:5:2, 2:5:3, 4:4:2, 3:4:3, 2:4:4, 6:3:1, 7:2:1, and 8:1:1, and preferably the weight ratio of sucrose to glycine to dextran is 5:4:1.

When the suspension comprises trehalose and mannitol, the weight ratio of trehalose to mannitol is suitably from 1:4 to 4:1, or from 1:2 to 2:1, e.g. 1:4, 3:2, 2:3, 4:1, 2:1, 1:2 or 1:1, and preferably the weight ratio is 1:1.

When the suspension comprises sucrose, mannitol and dextran, the weight ratio of sucrose to mannitol to dextran is suitably selected from 5:4:1, 4:5:1, 3:5:2, 2:5:3, 4:4:2, 3:4:3, 2:4:4, 6:3:1, 7:2:1, and 8:1:1, and preferably the weight ratio is 3:5:2.

When the suspension comprises trehalose, mannitol and dextran, the weight ratio of trehalose to mannitol to dextran is suitably selected from 5:4:1, 4:5:1, 3:5:2, 2:5:3, 4:4:2, 3:4:3, 2:4:4, 6:3:1, 7:2:1, or 8:1:1.

When the suspension comprises trehalose, glycine and dextran, the weight ratio of trehalose to glycine to dextran is suitably 5:4:1, 4:5:1, 3:5:2, 2:5:3, 4:4:2, 3:4:3, 2:4:4, 6:3:1, 7:2:1, and 8:1:1.

When the suspension comprises trehalose and glycine, the weight ratio of trehalose to glycine is suitably from 1:4 to 4:1, or from 1:2 to 2:1, e.g. 1:4, 3:2, 2:3, 4:1, 2:1, 1:2 or 1:1, and preferably the weight ratio is 1:1.

When the suspension comprises sucrose and glycine, the weight ratio of sucrose to glycine is suitably from 1:

4 to 4:1, or from 1:2 to 2:1, e.g. 1:4, 3:2, 2:3, 4:1, 2:1, 1:2 or 1:1, and preferably the weight ratio is 1:1.

When the suspension comprises mannitol and glycine, the weight ratio of mannitol to glycine is suitably from 1:4 to 4:1, or from 1:2 to 2:1, e.g. 1:4, 3:2, 2:3, 4:1, 2:1, 1:2 or 1:1, and preferably the weight ratio is 1:1.

The above mentioned suspensions may in addition contain 0.1-5 % by weight of a surfactant, e.g. a polyethylene glycol sorbitan monolaurate (Tween 20), a polyoxyethylene sorbitan monooleate (Tween 80), a block copolymer of polyethylene and polypropylene glycol (Pluronic).

According to one embodiment, the formulation according to the invention is a tablet.

The tablet may be a non-compressed tablet (e.g. a fast dissolving tablet).

According to another embodiment of the invention, the tablet is a compressed tablet.

Any of the tablets as above may be for sublingual administration.

The formulation according to the invention may also be a powder.

In a further embodiment of the invention, the formulation is in the form of particles.

Also, the formulation may be a granulate.

In a further embodiment, the formulation is a capsule comprising the lyophilisate. The lyophilisate contained in the capsule may be in the form of a powder, particles or a granulate. In one embodiment the capsule comprises coated microparticles as described below.

Antigen

The solid formulation according to the invention may contain one antigen, or alternatively more than one antigen.

Allergens

In a particular embodiment of the invention the antigen is an allergen. The allergen may be any naturally occurring protein that has been reported to induce allergic, i.e. IgE mediated reactions upon their repeated exposure to an individual. Examples of naturally occurring allergens include pollen allergens (tree-, herb, weed-, and grass pollen allergens), insect allergens (inhalant, saliva and venom allergens, e.g. mite allergens, cockroach and midges allergens, hymenopthera venom allergens), animal hair and dandruff allergens (from e.g. dog, cat, horse, rat, mouse etc.), and food allergens. Important pollen allergens from trees, grasses and herbs are such originating from the taxonomic orders of Fagales, Oleales, Pinales and platanaceae including i.a. birch (Betula), alder (Alnus), hazel (Corylus), hornbeam (Carpinus) and olive (Olea), cedar (Cryptomeria and Juniperus), Plane tree (Platanus), the order of Poales including i.a. grasses of the genera Lolium, Phleum, Poa, Cynodon, Dactylis, Holcus, Phalaris, Secale, and Sorghum, the orders of Asterales and Urticales including i.a. herbs of the genera Ambrosia, Artemisia, and Parietaria. Other important inhalation allergens are those from house dust mites of the genus Dermatophagoides and Euroglyphus, storage mite e.g Lepidoglyphys, Glycyphagus and Tyrophagus, those from cockroaches, midges and fleas e.g. Blatella, Periplaneta, Chironomus and Ctenocepphalides, and those from mammals such as cat, dog and horse, venom allergens including such originating from stinging or biting insects such as those from the taxonomic order of Hymenoptera including bees (superfamily Apidae), wasps (superfamily Vespidea), and ants (superfamily Formicoidae). Important inhalation allergens from fungi are i.a. such originating from the genera Alternaria and Cladosporium.

In a more preferred embodiment of the invention the allergen is Bet v 1, Aln g 1, Cor a 1 and Car b 1, Que a 1, Cry j 1, Cry j 2 , Cup a 1, Cup s 1, Jun a 1, Jun a 2, jun a 3, Ole e 1 , Lig v 1, Pla l 1, Pla a 2, Amb a 1, Amb a 2, Amb t 5, Art v 1, Art v 2 Par j 1 , Par j 2, Par j 3, Sal k 1, Ave e 1, Cyn d 1, Cyn d 7, Dac g 1, Fes p 1, Hol l 1, Lol p 1 and 5, Pha a 1, Pas n 1, Phl p 1, Phl p 5, Phl p 6, Poa p 1, Poa p 5, Sec c 1, Sec c 5, Sor h 1, Der f 1 , Der f 2, Der p 1, Der p 2, , Der p 7, Der m 1, Eur m 2, Gly d 1, Lep d 2, Blo t 1, Tyr p 2, Bla g 1, Bla g 2, Per a 1, Fel d 1, Can f 1, Can f 2 , Bos d 2, Equ c 1, Equ c 2, Equ c 3 , Mus m 1, Rat n 1, Apis m 1, Api m 2 , Ves v 1, Ves v 2, Ves v 5, Dol m 1, Dil m 2, Dol m 5, Pol a 1, Pol a 2, Pol a 5, Sol i 1, Sol i 2, Sol i 3 and Sol i 4, Alt a 1, Cla h 1, Asp f 1, Bos d 4, Mal d 1, Gly m 1, Gly m 2, Gly m 3, Ara h 1, Ara h 2, Ara h 3, Ara h 4, Ara h 5 or shufflant hybrids from Molecular Breeding of any of these.

In the most preferred embodiment of the invention the allergen is grass pollen allergen or a dust mite allergen or a ragweed allergen or a cedar pollen or a cat allergen or birch allergen.

Suitably the grass pollen allergen is selected from Phl p 1, Phl p 5 and Phl p. The birch pollen allergen is Bet v 1. The ragweed allergen is selected from Art v 1 and Art v 2.

In yet another embodiment of the invention the solid vaccine formulation comprises at least two different types of allergens either originating from the same allergic source or originating from different allergenic sources e.g. grass group 1 and grass group 5 allergens or mite group 1 and group 2 allergens from different mite and grass species respectively, weed antigens like short and giant ragweed allergens, different fungis allergens like alternaria and cladosporium, tree allergens like birch, hazel, hornbeam, oak and alder allergens, food allergens like peanut, soybean and milk allergens.

The allergen(s) incorporated into the solid vaccine formulation may be in the form of an extract, mixtures of extracts, a purified allergen, a modified allergen, a recombinant allergen or a mutant of a recombinant allergen. An allergenic extract may naturally contain one or more isoforms of the same allergen, whereas a recombinant allergen typically only represents one isoform of an allergen. In a preferred embodiment the allergen is in the form of an extract. In another preferred embodiment the allergen is a recombinant allergen. In a further preferred embodiment the allergen is a naturally occurring low IgE-binding mutant or a recombinant low IgE-binding mutant.

By recombinant allergen is meant an allergen produced by recombinant techniques using expression systems.

Allergens may be present in equi-molar amounts or the ratio of the allergens present may vary preferably up to 1:20.

In a further embodiment of the invention the low IgE binding allergen is an allergen according to WO 99/47680, WO02/40676 or WO 03/096869.

Microbial agents

In another particular embodiment of the invention the antigen is a microbial agent. In particular, the microbial agent is a virus, a bacterium, a fungus, a parasite or any part thereof.

Examples of microbial agents are Vibrio species, Salmonella species, Bordetella species, Haemophilus species, Toxoplasmosis gondii, Cytomegalovirus, Chlamydia species, Streptococcal species, Norwalk Virus, Escherischia coli, Helicobacter pylori, Helicobacter felis, Rotavirus, Neisseria gonorrhae, Neisseria meningiditis, Adenovirus, Epstein Barr Virus, Japanese Encephalitis Virus, Pneumocystis carini, Herpes simplex, Clostridia species, Respiratory Syncytial Virus, Klebsielia species, Shigella species, Pseudomonas aeruginosa, Parvovirus, Campylobacter species, Rickettsia species, Varicella zoster, Yersinia species, Ross River Virus, J. C. Virus, Rhodococcus equi, Moraxella catarrhalis, Borrelia burgdorferi, Pasteurella haemolytica, poliovirus, influenza virus, Vibrio cholerae and Salmonella enterica serovar Typhi.

Further examples of microbial agents are those, which prevent or reduce the symptoms of the following diseases: Influenza, Tuberculosis, Meningitis, Hepatitis, Whooping Cough, Polio, Tetanus, Diphtheria, Malaria, Cholera, Herpes, Typhoid, HIV, AIDS, Measles, Lyme disease, Travellers Diarrhea, Hepatitis A, B and C, Otitis Media, Dengue Fever, Rabies, Parainfluenza, Rubella, Yellow Fever, Dysentery, Legionnaires Disease, Toxoplasmosis, Q-Fever, Haemorrhegic Fever, Argentina Haemorrhagic Fever, Caries, Chagas Disease, Urinary Tract Infection caused by E. coli, Pneumoccoccal Disease, Mumps, and Chikungunya.

Oxygen-containing metal salt

Examples of suitable oxygen-containing metal salts are e.g. those, wherein the cation is selected from Al, K, Ca, Mg, Zn, Ba, Na, Li, B, Be, Fe, Si, Co, Cu, Ni, Ag, Au, and Cr.

The anion of the oxygen-containing compound may be an organic or inorganic anion, or a combination of organic and inorganic anions. Examples of suitable oxygen-containing metal salts are e.g. those, wherein the anion selected from sulphates, hydroxides, phosphates nitrates, iodates, bromates, carbonates, hydrates, acetates, citrates, oxalates, and tartrates, as well as mixed forms thereof. The oxygen-containing metal salts further comprise coordination complexes. A definition of coordination complexes is given in e.g. The Handbook of Chemistry and Physics 56 Ed., Section B, Chapter 7 (1975-76).

Within the present context, the expression "mixed forms" is intended to include combinations of the various anions as well as combinations with e.g. chlorides, and sulphides.

Although the solid vaccine formulation comprises an oxygen-containing metal salt, it is contemplated that the oxygen could be substituted by another Group VIA atom such as S, Se or Te.

The oxygen-containing metal salt to be used in accordance with the invention may be any oxygen-containing metal salt providing the desired effect when formulated into a mucosal delivery system. Examples of such oxygen-containing substances are aluminium hydroxide, aluminium phosphate, aluminium sulphate, potassium aluminium sulphate, calcium phosphate, Maalox (mixture of aluminium hydroxide and magnesium hydroxide), beryllium hydroxide, zinc hydroxide, zinc carbonate, zinc chloride and barium sulphate. Preferred oxygen-containing metal salts are aluminium hydroxide, aluminium phosphate and calcium phosphate. Most preferred the oxygen containing metal salt is aluminium hydroxide.

Preparation of the lyophilisate

The lyophilisate of a suspension of an oxygen-containing metal salt, excipient(s) and antigen may be prepared by initially forming a suspension of an oxygen-containing metal salt, excipients and antigen and optionally additional matrix-forming agent and/or other agents. Such additional matrix-forming agents and/or other agents are selected with due regard to the type of formulation to be made, cf. below. The suspension is preferably an aqueous suspension.

When using e.g. aluminium hydroxide as oxygen-containing metal salt, the concentration of aluminium hydroxide (as mg Al/ml) in the liquid suspension is preferably 0.035-1000 mg/ml, more preferably 0.10-100 mg/ml, and most preferably 2-30 mg/ml For the other oxygen-containing metal salts, the concentration of the metal salt (as mg cation/ml) is preferably 0.035-1000 mg/ml, more preferably 0.35-100 mg/ml, more preferably 0.7-75 mg/ml, and most preferably 1.5-50 mg/ml.

When the antigen is an allergen the concentration of allergen in the suspension is preferably 0.01-100 mg/ml, more preferably 0.1-10 mg/ml. The weight ratio of oxygen-containing metal salt to allergen is preferably from 0.1 to 100, more preferably from 1 to 20. The degree of allergen adsorbed to the oxygen-containing metal salt is typically from 5 to 99 %, more preferably from 10 to 99 % of the added amount. The adsorption of allergen to the oxygen-containing metal salt depends on the buffer system and the reaction conditions, including temperature and reaction time, under which the adsorption takes place.

The pl of the oxygen-containing metal salt is typically in the range of 2-11. The pl for allergen proteins is typically in the range of 4-9. Preferably, the allergen and oxygen-containing metal salt are selected so that the pl of the allergen is lower than the pl of the oxygen-containing metal salt.

The total concentration of excipients in the suspension is preferably from 0.1 mM to 1.2 M, more preferably from 0.5 mM to 800 mM, and most preferably from 100 to 500 mM.

The suspension preferably comprises 5-25 % by weight of excipients (total amount) or more preferred 10-15 % by weight of excipients (total amount).

The suspension is then subjected to lyophilisation. The lyophilisation method and technology is selected with due regard to the type of solid formulation to be prepared, e.g. a powder, particles or a granulate.

In one embodiment of the invention the suspension is divided into drops and freezed to obtain cryodrops, which are subsequent subjected to lyophilisation to obtain granules. In another embodiment of the invention a suitable volume of the suspension is placed in a container and lyophilised to obtain a cake residue, which is then milled to form particles or a powder of particles having a desired size.

In yet another embodiment of the invention, the suspension is loaded into depressions of a multilayer laminated blister sheet, and the loaded sheet is subjected to freeze-drying to form non-compressed tablets. Then the sheet is optionally sealed.

In yet another embodiment, the suspension is subjected to spray-freeze-drying as described in US 2003/0202978, optionally using lower solids concentration in the suspension, or as described in US 2005/0266021.

Solid vaccine formulations for mucosal administration

The solid vaccine formulation of the invention may be administered via any mucosa, including the oral (via the mucosa of the digestive system, e.g the gastrointestinal mucosa), nasal, vaginal, sublingual, ocular, rectal, urinal, intramammal, pulmonal, otolar (i.e. via the ear), buccal or oromucosal mucosa, preferably via the buccal or sublingual mucosa, or the gastrointestinal mucosa.

It has been speculated that it is preferable to carry out a mucosal administration of a vaccine via the mucosa, which is subject to the natural exposure to the antigen. Accordingly, for allergies to airborne mucosal antigens, it may be preferred to use administration via the respiratory system, preferably a buccal or sublingual administration. Correspondingly, for allergies to mucosal agents, which comes into contact with the mucosa of the digestive system, it may be preferred to use oral administration.

The solid vaccine formulation of the invention may have any solid form, which is suitable for mucosal administration, and which consists of or comprises a lyophilisate, including formulations selected form the group consisting of a powder, particles, granules, a compressed tablet, a non-compressed tablet, an implant, a capsule having a single wall and containing lyophilisate, e.g. in the form of powder, particles or granules, and a microparticle having a coherent encapsulating agent and lyophilisate in the form of powder, particles or granules embedded therein. The formulation may also be a capsule (e.g. a gelatine capsule) comprising the lyophilisate in the form of powder, a granulate, particles or in the form of encapsulated microparticles as above.

Compressed tablets

The lyophilisate, e.g. in the form of a powder, particles or granules, may be used to prepare a compressed tablet. The compressed tablet may include any conventional tablet-forming agent or excipients. The table may also contain the lyophilisate in the form of microparticles having a coherent encapsulating agent.

Non-compressed tablets

In a particular embodiment of the invention, the solid vaccine formulation is a fast-dissolving, non-compressed tablet suitable for buccal or sublingual administration. Examples of fast dissolving, non-compressed tablets are those disclosed in US-A-5,648,093, WO 00/51568, WO 02/13858, WO99/21579, WO 00/44351, US-A-4,371,516, EP-278 877, WO 2004/047794 and WO 2004/075875. Preferred fast dissolving, non-compressed tablets are those produced by freeze-drying. Preferred matrix forming agents are fish gelatine and modified starch.

The non-compressed tablet may in addition to the lyophilisate, include any conventional tablet-forming agent or excipient, such as adjuvants, antacids, diluents, enhancers, mucoadhesive agents, flavouring agents, taste masking agents, preservatives, antioxidants, surfactants, viscosity enhancers, colouring agents, pH modifiers, sweeteners etc. These excipients are all selected in accordance with conventional pharmaceutical practice in a manner understood by the persons skilled in the art of formulating allergen vaccines.

In a preferred embodiment of the invention, the tablet contains a protein stabilizing agent. Examples of protein stabilising agents are polyethylene glycols (PEG), e.g. PEG300, PEG400, PEG600, PEG1000, PEG1500, PEG3000, PEG3050, PEG4000, PEH6000, PEG20000 and PEG35000; amino acids, such as glycine, alanine, arginine; mono-, di and trisaccharides, such as trehalose and sucrose; polyvinylalcohol (PVA); polyoxyethylene sorbitan fatty acid esters (polysorbates, tweens or span); human serum albumin (HSA); bovine serum albumin (BSA). Preferably, PEG is used as protein stabilising agent. In addition to being a protein stabiliser, PEG is believed to confer the property of elasticity to the matrix of the dosage form.

Suitable colouring agents include red, black and yellow iron oxides and FD & C dyes such as FD & C blue No. 2 and FD & C red No. 40. Suitable flavouring agents include mint, raspberry, liquorice, orange, lemon, grapefruit, caramel, vanilla, cherry and grape flavours and combination of these. Suitable pH modifiers include citric acid, tartaric acid, phosphoric acid, hydrochloric acid and maleic acid. Suitable sweeteners include aspartame, acesulfame K and thaumatic. Suitable taste-masking agents include sodium bicarbonate, ion-exchange resins, cyclodextrin inclusion compounds, adsorbates or microencapsulated actives.

Adjuvants are normally used to enhance the absorption of the antigen (e.g. allergen) as well as to enhance the immune-stimulating properties of the antigen (e.g. allergen).

In one embodiment of the invention at least one adjuvant in addition to the oxygen-containing metal salt is incorporated into the tablet according to the invention. Examples of suitable adjuvants are heat-labile enterotoxin (LT), cholera toxin (CT) (and detoxified fractions thereof), cholera toxin B (CTB), polymerised liposomes, mutant toxins, e.g. LTK63 and LTR72, microcapsules, non-toxic bacterial fragments, cytokines, chitosan, homologous heat-labile of E.coli (and detoxified fractions thereof), saponins, bacterial products such as lipopoly-saccharides (LPS) and muramyl dipeptide (MDP), liposomes, CpG (immunostimulatory DNA sequences), lactide/glycolide homo (copolymers in the form of microparticular polymers etc. The use of adjuvants in allergen vaccines are often reasoned by the fact the allergens in question are not able to penetrate the barrier to be passed. The adjuvants thus may serve as absorption enhancing agents or they may act as immunostimulants.

The non-compressed fast dissolving tablet according to the invention may be mucoadhesive to some extent in itself; however in a preferred embodiment of the invention, it may be desirable to further add mucoadhesive excipients to said dosage form in order to increase the contact time of the dosage form with the mucosa of the oral cavity. Suitable mucoadhesive excipients are polyacrylic polymers such as carbomer and carbomer derivatives; cellulose derivatives such as hydroxypropylmethylcellulose, hydroxyethylcellulose, hydroxypropylcelllulose and sodium carboxymethylcellulose; natural polymers such as gelatine, sodium alginate, pectin and glycerol.

Microparticles

Peyer's patches are aggregates of lymphoid nodules located in the wall of the small intestine, large intestine and appendix and are an important part of body's defense against the adherence and penetration of infection agents and other substances foreign to the body. Peyer's patches are also known as folliculi lymphatic aggregati. Similar folliculi lymphatic aggregati can be found in the respiratory tract, the rectum, the nasal cavity, the oral cavity, the pharynx, the genitourinary tract, large intestine and other mucosal tissues of the body. The said tissues may in general be referred to as mucosally-associated lymphoid tissues (MALT).

It has been shown that pharmaceutically active substances formulated as microparticles having a proper size and suitable physico-chemical properties may be effectively taken up by Peyer's patches and MALT.

The use of microparticles involves the advantage of protecting the pharmaceutical active substance from degradation, both during production and storage of the dosage forms, and in the process of administration of the active substance to the patient. This is particularly important, when the active substance is an allergen. The use of microencapsulation to protect sensitive bioactive substances from degradation has become well-known. Typically, a bioactive substance is encapsulated within any of a number of protective wall materials, usually polymeric in nature. The amount of substance inside the microparticle can be varied as desired, ranging from either a small amount to as high as 95% or more of the microparticle composition. The diameter of the microparticle is preferably less than 20 $\mu$m, more preferably less than 15 $\mu$m, more preferably less than 10 $\mu$m and most preferably between 1 and 10 $\mu$m.

The encapsulating agent may be any biodegradable agent, preferably a polymeric agent. Preferably, the encapsulating agent is selected from the group consisting of poly-lactide, poly-lactid-poly(ethylene glycol), poly(DL-lactide-co-glycolide), poly(glycolide), copolyoxalates, polycaprolactone, poly(lactide-co-caprolactone), poly(esteramides , polyorthoesters and poly(8-hydroxybutyric acid), and polyanhydrides, most preferably poly(DL-lactide-co-glycolide). Other examples of encapsulating agents are poly(butyl-2-cyanoacrylate), poly(3-hydroxybutyrate) and polyanhydride copolymers of fumaric and sebacic acid, poly(FA:SA). Also, suitable encapsulating agents for use according to the present invention include those derived from animal or vegetable proteins, such as gelatines, dextrins and soy, wheat and psyllium seed proteins; gums such as acacia, guar, agar and xanthan; polysaccarides; starch and modified starch, alignates; carboxymethylcellulose; carrageenans; dextrans; pectins; synthetic polymers such as polyvinylpyrrolidone; and polypeptide/protein or polysaccharide complexes such as gelatine-acacia complexes. In one embodiment of the invention two or more encapsulating agents are used. Preferably, the encapsulating agent is selected so as to make the microparticles hydrophobic. It is believed that hydrophobic microparticles are more easily taken up by the MALT or allowed to elicit its effects via the MALT.

In one embodiment, the encapsulating agent used is an enteric coating of a ethylacrylate methacrylic acid copolymer, hydroxypropylmethylcellulose acetate succinate, or other enteric coating, see e.g. US patent No. 5,591,433.

Capsules

The capsules of the invention may be any conventional type of capsule. In a particular embodiment of the invention, the capsule wall is composed of gelatine. In particular, the capsule may be adapted for oral administration. The capsule wall may be composed of or coated with any of the encapsulating agents mentioned above in connection with microparticles.

According to the invention, the capsules may contain the lyophilisate of a suspension comprising antigen, oxygen containing metal salt and excipients, in the form of a powder, granules or particles. Alternatively, the capsule may contain the microparticles as described above.

Buccal or sublingual treatment

The solid vaccine formulation may be used in a vaccination protocol comprising daily administration of the vaccine. Another vaccination protocol comprises administration of the vaccine every second day, every third day or every fourth day. For instance, the vaccination protocol comprises administration of the vaccine for a period of more than 4 weeks, preferably more than 8 weeks, more preferably more than 12 weeks, more preferably more than 16 weeks, more preferably more than 20 weeks, more preferably more than 24 weeks, more preferably more than 30 and most preferably more than 36 weeks.

The period of administration may a continuous period. Alternatively, the period of administration is a discontinuous period interrupted by one or more periods of non-administration. Preferably, the (total) period of non-administration is shorter than the (total) period of administration.

The vaccine may be administered to the test individual once a day. Alternatively, the vaccine is administered to the test individual twice a day. The vaccine may be a uni-dose vaccine. Alternatively, the vaccine is a multidose vaccine.

Classical incremental dosage desensitisation treatment, where the dose of allergen in the form of a solid formulation is increased to a certain maximum, relieves the symptoms of allergy. The preferred potency of a unit dose of the tablet is from 150 — 1000000 SQ-u/tablet, more preferred the potency is from 500 — 500000 SQ-u/tablet and more preferably the potency is from 1000 — 250000 SQ-u/tablet, even more preferred 1500-125000 SQ-u/tablet most preferable 1500-75000 SQ-u/tablet.

In another embodiment of the invention the tablet is a repeated mono-dose, preferably within the range of 1500-75000 SQ-u/tablet.

DEFINITIONS

In connection with the present invention the following definitions are used:

The terms "lyophilisation" and "freeze-drying" are used interchangeably.

The term "lyophilisate" means any form of solid residue resulting from a lyophilisation process, e.g. in the form of a cake or a granulate or a powder.

The term "oromucosal" means relating to the mucosa of the oral cavity, the mucosa of the pharynx and the sublingual mucosa.

The term "buccal" means relating to the oral cavity.

The term "sublingual" means relating to the position below the lingua of the oral cavity.

The term "SQ" means SQ-Unit: The SQ-Unit is determined in accordance with ALK-Abelló A/S's "SQ biopotency"-standardisation method, where 100,000 SQ units equal the standard subcutaneous maintenance dose. Normally 1 mg of extract contains between 100,000 and 1,000,000 SQ-Units, depending on the allergen source from which they originate and the manufacturing process used. The precise allergen amount can be determined by means of immunoassay i.e. total major allergen content and total allergen activity.

EXAMPLES

EXAMPLE 1: Spray freeze drying of a suspension comprising aluminium hydroxide and various excipients

The composition of the suspensions subjected to spray-freeze-drying (SFD) were as shown in table 1

Table 1: Formulations for spray freeze-drying. Mw for dextran is 70.000 dalton

| Formulation | Composition | Solid content before SFD | Aluminum content (mg/ml in liquid formulation) |
|---|---|---|---|
| Sucrose/mannitol | 1:1 | 10 % | 1.17 |
| Sucrose/Glycine/ dextran | 5:4:1 | 10 % | 1.17 |
| Sucrose/Mannitol | 1:1 | 10 % | 2.34 |
| Trehalose/Mannitol | 1:1 | 10% | 2.34 |
| Sucrose/Mannitol | 1:1 | 15 % | 2.34 |
| Trehalose/Mannitol | 1:1 | 15 % | 2.34 |
| Sucrose/Mannitol | 1:1 | 15 % | 2.34 |
| Sucrose/Mannitol/ dextran | 3:5:2 | 15 % | 2.34 |

The formulations were spray frozen using the system: Freeze granulator LS 2 (PowderPro AB) and were dried using a Lyovac GT-2 freeze dryer (PowderPro) or a Usifroid freeze dryer (FRD0001 at ALK-Abe116). The system compromises a two-fluid nozzle held above a glass beaker standing on a magnetic stirrer. To reduce loss of liquid nitrogen a cover is placed on top of the glass beaker. The beaker was approximately half filled with liquid nitrogen. A pause of 2-3 minutes was allowed for stabilization of the liquid nitrogen surface. The formulation was sprayed into the liquid nitrogen using a feed rate of 4 1/hour (for most formulations) controlled by a peristaltic pump. An air pressure in the range 0.15 bar- 0.3 bar were used during spraying. The dispersion of frozen particle produced was continuously stirred and transferred to freeze drying trays topped with liquid nitrogen. Drying at PowderPro was done in a Lyovac GT-2 freeze dryer at the lowest possible pressure (corresponds to approximately -33°C). Drying at ALK-Abe116 was performed in a Usifroid freeze dryer (FRD0001) using the settings listed in Table 2.

Table 2: Settings for Usifroid freeze dryer (FRD0001)

| Sequence | Shelf temperature °C | Pressure μbar | Time minutes |
|---|---|---|---|
| Shelf pre-cooling | -50 | - | - |
| Freeze 1 | -50±5 → -55±5 | - | 5 |
| Freeze 2 | -55±5 | - | 15 |
| Transition freezing—condenser cooling | -55±5 | - | 120 |
| Condenser temp. | -40°C | | |
| Primary 1[1] | -55±5 → -10±5 | 130 | 45 |
| Primary 2 | -10±5 | 130 | 1440 |
| Secondary 1 | -10±5 → 20±5 | 130 | 30 |

(continued)

| Sequence | Shelf temperature °C | Pressure µbar | Time minutes |
|---|---|---|---|
| Secondary 2 | 20±5 | 130 | 1440 |

Control of the powders consisted of visual inspection, flowability, reconstitution and bulk density. Alhydrogel characteristics were measured by sedimentation test and microscopic analysis (optic and polarised light). Acceptable powders were prepared using all the excipient compositions listed in table 1, though some powders tended to absorb moisture which resulted in stickiness/large particle agglomerates. The spray freeze-drying process in the presence of the listed excipients was not detrimental to Alhydrogel. Minor changes in sedimentation pattern were observed, however no large scale coagulation was observed using microscopic analysis.

EXAMPLE 2: Lyophilisation of an aluminium hydroxide-based vaccine formulation

Background and Aim

Aluminium hydroxide (Al(OH)3) based vaccine formulations must be stored refrigerated and under non-freezing conditions if the preparation are to maintain its potency and properties. Further, lyophilisation of a vaccine formulation reduce both the potency and the general properties (sedimentation velocity, fluidity) of the vaccine after reconstitution. Trehalose is a di-glucose carbon hydrate, which have been used as an additive to stabilise various compounds during lyophilisation and storage. The aim of the present investigations was to determine the effects of trehalose, in particular during lyophilisation, on a vaccine formulation in the form of a mixture of an aqueous suspension of aluminium hydroxide and an extract of the allergen Der p or Phl p, in particular the effects on the physical and chemical properties, the activity / potency, and the immunogenicity of the vaccine.

Methods

Sample preparation

In all analysis carried out on aluminium hydroxide alone a 20% of Alhydrogel ® 1.3 % obtained from Brenntag (content of ash residue (Al2O3): 1.3 % w/w; content of corresponding Al(OH)3: 1.99 % w/w; content of aluminium: 6.25 mg Al/ml ± 5%) has been used.

Adsorption

The liquid formulations were prepared by mixing over night at 4-8 °C 10% of allergen, 1000000 SQ/ml with 20% of Alhydrogel ® 1.3 % obtained from Brenntag (content of ash residue (Al2O3): 1.3 % w/w; content of corresponding Al(OH)3: 1.99 % w/w; content of aluminium: 6.25 mg Al/ml ± 5%). Additional 70% Coca's buffer with or without trehalose was added. In one case FITC labelled Phl p extract was used in adsorption.

Lyophilisation

Samples of 1 mL (1.2 mL for the mouse immunisation protocol) were aliquoted into glass vials. The samples were then frozen at -80°C for 5 min and then lyophilised over night. The lyophlised powder was studied before the samples were re-dissolved in 1 mL MilliQ water.

Microscope analysis

The samples were analysed using Leica TCS SP2/Leica DC500 for both phase contrast microscope and fluorescence microscope analysis. The samples were also analysed with a polarisation microscope (Olympus SZ 60).

Rocket Immunoelectrophoresis RIE

RIE was performed according to the protocol described in Axelsen et al. 1973

[1]. In RIE the sample is separated electrophoretically in one dimension in an agarose gel. The identification of the protein is in RIE given by a rocket shaped precipitate. For the Phl p vaccine RIE with anti Phl p and anti Phl p 6 raised against the supernatant of the samples were run. For the Der p vaccine RIE with anti Der p, anti Der p 1 and anti Der p 2 raised against the supernatant of the samples were run.

IgE inhibition experiments

The IgE inhibition experiments were performed on an Advia Centaur system. A serum sample (25 μL of a serum pool obtained from mixing equal amounts of serum from house dust mite allergic individuals) was mixed with a solid phase (PMP, paramagnetic particles) immobilized anti-IgE antibody. After incubation and washing, serial dilutions of the inhibitor (extract, vaccine or vaccine lyophilized and re-dissolved) were mixed with a constant amount of biotinylated Der p. The amount of biotinylated Der p bound to the solid phase adsorbed IgE antibodies was estimated as the amount of light emitted (RLU) after addition of acridinium ester labelled Streptavidin, incubation and washing.

The RLU values measured was transformed into the degree of binding (DoB) according to equation 1.

$$DoB = \frac{RLU_i - RLU_{BasePool}}{RLU_{i=0} - RLU_{BasePool}} \qquad \text{Eqn. 1,}$$

wherein RLUi is the readout obtained in the presence of inhibitor i at a given dose, RLUBasePool is the background signal and RLUi=0 is the signal obtained using buffer as inhibitor (0% inhibition). All RLU values were determined as triplicate dependent measurements and the mean of these was used in the calculations.

Data sets of (log10Dose,DoB) for each tested inhibitor preparation was fitted (GrapPad Prism v. 4.03) to a logistic function according to equation 2.

$$DoB = B + \frac{T - B}{1 + 10^{(\log EC50 - \log Dose)*HS}} \qquad \text{Eqn. 2,}$$

wherein B, T, logEC50 and HS are parameters determined from the nonlinear fit. B is the "bottom" asymptote of the S-shaped curve, T is the "top" asymptote of the S-shaped curve, EC50 is the dose needed to obtain 50% inhibition and HS (Hill Slope) is the estimator of the steepness or "slope" of the S-shaped curve. The HS values were compared directly in the fitting procedure and if the slopes of the compared curves were statistically indistinguishable, logEC50 values based upon a common slope estimate was calculated and used in the potency comparison. All statistical calculations were performed with GraphPad Prism v. 4.03.

The inhibition experiment was performed on two separate days and the data was pooled before analysed.

Mouse immunisations with formulations containing trehalose - Immunisation Protocol

Mice where immunised i.p. (interperitoneally) every two weeks in a total of 6 times, with 100 μl liquid formulation having one of the following compositions:

1. Phl p (Phleum pratense extract) coupled to Alhydrogel (Phl p Alum).
2. Phl p coupled to Alhydrogel, freeze dried and re-dissolved immediately before use (FD Phl p Alum).
3. Phl p + 100 mM trehalose coupled to Alhydrogel (Phl p TH100).
4. Phl p + 200 mM trehalose coupled to Alhydrogel (Phl p TH200).
5. Phl p + 300 mM trehalose coupled to Alhydrogel (Phl p TH300).
6. Phl p + 100 mM trehalose coupled to Alhydrogel, freeze dried and re-dissolved immediately before use (FD Phl p TH100).
7. Phl p + 200 mM trehalose coupled to Alhydrogel, freeze dried and re-dissolved immediately before use (FD Phl p TH200).
8. Phl p + 300 mM trehalose coupled to Alhydrogel, freeze dried and re-dissolved immediately before use (FD Phl p TH300).
9. Der p (Dermatophagoides pteronyssinus extract) coupled to Alhydrogel (Der p Alum).
10. Der p coupled to Alhydrogel, freeze dried and re-dissolved immediately before use (FD Der p Alum).
11. Der p + 100 mM trehalose coupled to Alhydrogel (Der p TH100).

12. Der p + 200 mM trehalose coupled to Alhydrogel (Der p TH200).

13. Der p + 300 mM trehalose coupled to Alhydrogel (Der p TH300).

14. Der p + 100 mM trehalose coupled to Alhydrogel, freeze dried and re-dissolved immediately before use (FD Der p TH100).

15. Der p + 200 mM trehalose coupled to Alhydrogel, freeze dried and re-dissolved immediately before use (FD Der p TH200).

16. Der p + 300 mM trehalose coupled to Alhydrogel, freeze dried and re-dissolved immediately before use (FD Der p TH300),

Each group contained 7-8 mice, Balb c/A where used for group 1-8 and SJL for group 9-16.

The liquid formulations where prepared by mixing over night at 4-8 °C 10% of allergen, 1000000 SQ/ml with 20% of Alhydrogel ® 1.3 % obtained from Brenntag (content of ash residue (Al2O3): 1.3 % w/w; content of corresponding Al(OH)3: 1.99 % w/w; content of aluminium: 6.25 mg Al/ml $\pm$ 5%). Additional 70% Coca's buffer with or without trehalose was added. Part of each formulation was lyophilised as described above in methods of lyophilisation. The lyophilised samples were stored in a freezer and re-dissolved prior to each immunisation.

Blood samples were taken after 0, 2, 3, 5 and 6 immunisations, serum collected and the level of allergen specific IgG determined by the following assay.

IgG assay

Phl p, Der p 1 or Der p 2 specific IgG was determined in group 1-8 and 9-16 respectively by a direct ELISA. The ELISA was performed by coating with allergen, blocking, incubating with serum and detection by HRP labelled antimouse IgG followed by TMB and acidic acid to stop the reaction. Absorbance was measured on an ELISA reader. Each step was separated by a wash.

Results and Discussion

Microscope analysis and RIE

First the effect of trehalose on aluminium hydroxide without antigen was tested.

Phase contrast images of untreated aluminium hydroxide stored at 5°C without trehalose and with 300 mM trehalose, and of lyophilised and re-dissolved aluminium hydroxide without trehalose and with 300mM trehalose show that trehalose does not seem to affect the aluminium hydroxide particles when the sample is stored at 5°C. When aluminium hydroxide is lyophilised platelet crystals are formed even after re-dissolving the sample. It is further seen that the lyophilised and re-dissolved aluminium hydroxide with 300 mM trehalose seem to have maintained the same gel structure as the reference samples stored at 5°C have.

Polarisation microscope images of the lyophilised and re-dissolved aluminium hydroxide without trehalose and with 300 mM trehalose clearly show that crystals are formed in the lyophilised re-dissolved aluminium hydroxide without trehalose, but that no crystals are visible when aluminium hydroxide is lyophilised in the presence of trehalose.

Phase contrast images of FITC-labelled aluminium hydroxide Phl p stored at 5°C with concentrations of trehalose of 0, 50, 100, 150, 200 and 300 mM, and of FITC-labelled aluminium hydroxide Phl p lyophilised and re-dissolved with concentrations of trehalose of 0, 50, 100, 150, 200 and 300 mM, show that with increasing trehalose concentration the structures of the gel particles in the re-dissolved samples increasingly maintain the gel structure of the reference samples. From the phase contrast images it seems that a trehalose concentration of 150-200mM is sufficient to maintain the gel structure.

Polarisation microscope images of aluminium hydroxide Phl p stored at 5°C with concentrations of trehalose of 0, 50, 100, 150, 200 and 300 mM, and of aluminium hydroxide Phl p lyophilised and re-dissolved with concentrations of trehalose of 0, 50, 100, 150, 200 and 300 mM, show that it is sufficient to lyophilise aluminium hydroxide Phl p with 50mM to prevent the formation of crystals.

RIE carried out with A) test samples in the form of the supernatant of aluminium hydroxide Phl p, which has been i) lyophilised and re-dissolved and ii) stored at 5 °C, for trehalose concentrations of 0 and 300 mM, precipitated by rabbit anti-Phl p antibodies, B) test samples in the form of standard solutions of Phl p containing 25000 SQ and 50000 SQ precipitated by rabbit anti-Phl p 6 antibodies, C) test samples in the form of the supernatant of aluminium hydroxide Phl p stored at 5 °C with trehalose concentrations of 0, 50, 100, 150, 200 and 300 mM, precipitated by rabbit anti-Phl p 6 antibodies, and D) test samples in the form of the supernatant of aluminium hydroxide Phl p lyophilised and re-dissolved with trehalose concentrations of 0, 50, 100, 150, 200 and 300 mM, precipitated by rabbit anti-Phl p 6 antibodies, show that the presence of trehalose increases the amount of Phl p and Phl p 6 in the supernatant signifying that the presence of trehalose desorbs some Phl p/Phl p 6. It is also observed that the desorption of Phl p 6 only increases slightly when increasing the trehalose concentration from 50mM to 300mM.

FITC-labelled aluminium hydroxide Phl p was lyophilised and re-dissolved without trehalose and with 300mM trehalose to find out whether Phl p extract is adsorbed to the aluminium hydroxide gel particles visualised by phase contrast microscopy. The samples were analysed by both phase contrast microscopy and fluorescence microscopy after which a superposition of the two images were formed. It appears that the fluorescence detected from the FITC-labelling has the same position as the aluminium hydroxide gel particles both for the lyophilised sample without and with 300mM trehalose indicating that the Phl p extract is adsorbed to the aluminium hydroxide gel particles. Furthermore, it appears that that gel particles lyophilised in the presence of trehalose are much smaller, homogenously distributed and resemble gel particles not subjected to lyophilisation as compared to the gel particles lyophilised in the absence of trehalose.

From phase contrast microscopy images of untreated aluminium hydroxide Der p stored at 5°C without trehalose and with 300 mM trehalose, and of lyophilised and re-dissolved aluminium hydroxide Der p without trehalose and with 300mM trehalose it appears that the presence of trehalose does not seem to affect the aluminium hydroxide gel particles stored at 5°C, but trehalose clearly stabilises the aluminium hydroxide Der p during lyophilisation as the gel particles maintain the same structure as the reference formulation stored at 5°C.

Polarisation microscopy images of untreated aluminium hydroxide Der p stored at 5°C without trehalose and with 300 mM trehalose, and of lyophilised and re-dissolved aluminium hydroxide Der p without trehalose and with 300mM trehalose also clearly show that crystals are formed when aluminium hydroxide Der p is lyophilised without trehalose, and no crystals are visualized when aluminium hydroxide Der p is lyophilised in the presence of 300mM trehalose.

From RIE carried out with A) test samples in the form of the supernatant of aluminium hydroxide Der p, which has been i) lyophilised and re-dissolved and ii) stored at 5 °C, for trehalose concentrations of 0 and 300 mM, precipitated by rabbit anti-Der p antibodies, B) test samples in the form of standard solutions of Der p 1 containing 33000 SQ, 100000 SQ and 300000 SQ and the supernatant of aluminium hydroxide Der p, which has been i) lyophilised and re-dissolved and ii) stored at 5 °C , for trehalose concentrations of 0 and 300 mM, precipitated by rabbit anti-Der p 1 antibodies, and C) test samples in the form of standard solutions of Der p 1 containing 33000 SQ, 100000 SQ and 300000 SQ and the supernatant of aluminium hydroxide Der p, which has been i) lyophilised and re-dissolved and ii) stored at 5 °C for trehalose concentrations of 0 and 300 mM, precipitated by rabbit anti-Der p 2 antibodies it appears that the presence of trehalose does not seem to induce any desorption.

IgE inhibition experiments

Vaccine formulations containing aluminium hydroxide as adjuvant must be kept above the freezing point in order to prevent irreversible damage to the formulation and it is not possible to lyophilize vaccine preparations without seriously compromising the activity of the vaccine. Trehalose is known to possess protein stabilizing properties and as demonstrated below this compound is able to stabilize aluminium hydroxide formulations under freeze drying conditions.

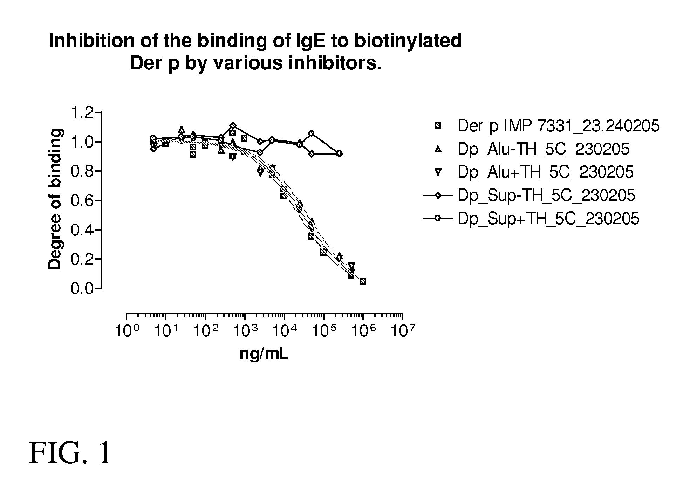

Fig. 1 shows the degree of inhibition of the binding of Der p-specific IgE to biotinylated Der p by various inhibitors, viz. Der p extract solution (Der p IMP 7331_23,240205), aluminium hydroxide Der p (Dp_Alu-TH_5C_230205), aluminium hydroxide Der p with 300 mM trehalose (Dp_Alu+ TH_5C_230205), supernatant of aluminium hydroxide Der p (Dp_Sup-TH_5C_230205), and supernatant of aluminium hydroxide Der p with 300 mM trehalose (Dp_Sup+TH_5C_230205). Fig. 1 illustrates that Trehalose (300 mM) can be mixed with an aluminium hydroxide formulation without any detectable change in the vaccine activity and no activity can be detected in the supernatant indicating that trehalose did not elute any of the aluminium hydroxide bound/absorbed proteins.

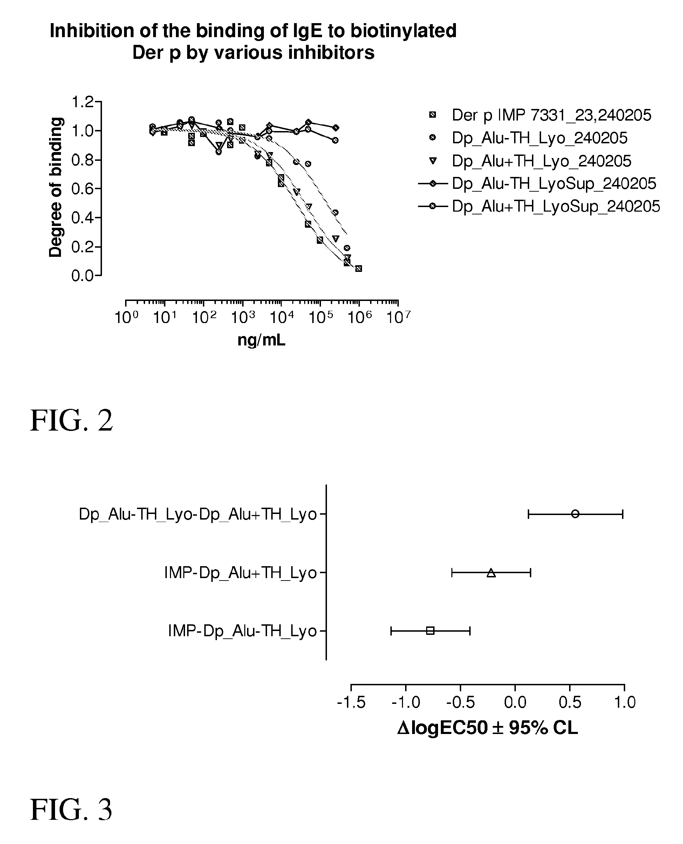

Fig. 2 shows the degree of inhibition of the binding of Der p-specific IgE to biotinylated Der p by various inhibitors, viz. Der p extract solution (Der p IMP 7331_23,240205), aluminium hydroxide Der p lyophilised (Dp_Alu-TH_Lyo_230205), aluminium hydroxide Der p with 300 mM trehalose (Dp_Alu+ TH_Lyo_230205), supernatant of aluminium hydroxide Der p (Dp_Sup-TH_LyoSup_230205), and supernatant of aluminium hydroxide Der p with 300 mM trehalose (Dp_Sup+TH_LyoSup_230205).

Fig. 2 shows that trehalose is able to maintain the potency of an aluminium hydroxide vaccine formulation after lyophilization and reconstitution in buffer. If trehalose is omitted in the lyophilization process the vaccine loses 85% of its former potency.

Fig. 3 shows a comparison of potency estimates for three test pairs of formulations, viz. i) Dp_Alu-TH_Lyo vs. Dp_Alu+TH_Lyo, ii) IMP vs. Dp_Alu+TH_Lyo, and iii) IMP vs. Dp_Alu-TH_Lyo using the denominations used above. The difference of the LogEC50 values including the 95 % Confidence Limit (CL) are plotted for the various test pairs. If the CL of the test pair includes 0, the values are statistically indistinguishable, and if it does not include 0 the values are statistically significantly different. As will appear from Fig. 3 the lyophilized and re-dissolved aluminium hydroxide preparation containing trehalose is statistically indistinguishable from the untreated reference preparation, whereas the preparation lyophilized without stabilizing agent significantly loses potency.

Mouse immunisation

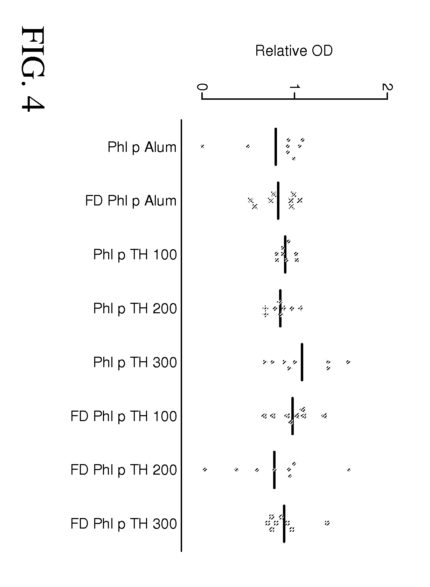

Fig. 4 shows the level of Phl p specific IgG in mice immunised three times with different Phl p formulations, wherein the Phl p specific IgG is depicted as the relative OD-value (the OD-value of the serum sample at the dilution 1: 64000 corrected with the OD-value for a reference sample at the dilution 1:40000) with each point representing one mouse serum. "Phl p alum" means aluminium hydroxide Phl p, FD means lyophilised and TH 100, 200 and 300 means trehalose 100, 200 and 300 mM, respectively. The results reveal that trehalose in itself does not inhibit the immune response towards Phl p in any of the concentrations used. No significant reduction of the immune response for aluminium hydroxide Phl p lyophilised in the absence of trehalose is observed as expected.

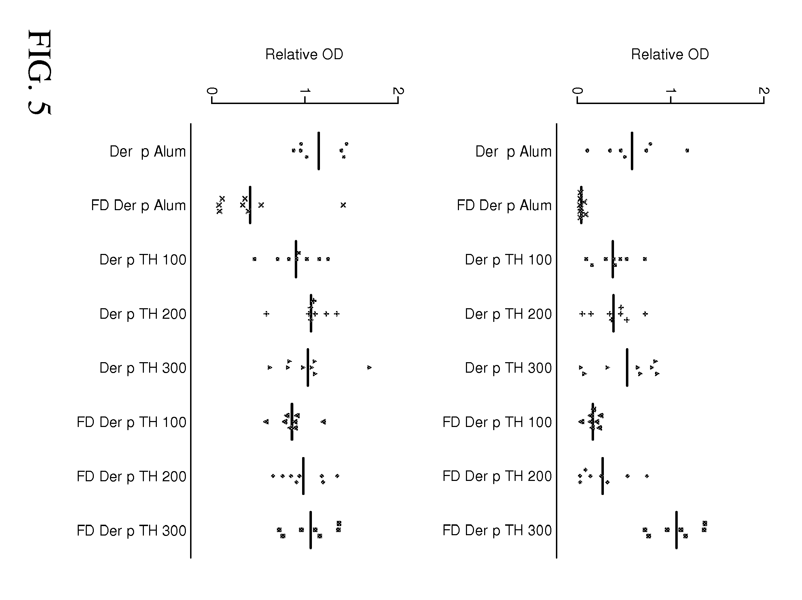

Fig. 5 shows the level of Der p 1 specific IgG in mice immunised two times (top panel) and five times (top panel) with different Der p formulations, wherein the Der p 1 specific IgG is depicted as the relative OD-value (the OD-value of the serum sample at the dilution 1:15000 corrected with the OD-value for a reference sample at the dilution 1:45000) with each point representing one mouse serum.

The results for mice immunised with different formulations of Der p also show, in consistency with the data for Phl p formulations, that trehalose does not inhibit the allergen specific immune response (Fig. 5, both panels). A lyophilised formulation of Der p without trehalose has a delayed and impaired response compared to a non-lyophilised formulation (Fig. 5, top and bottom panel), while addition of trehalose to the formulation protects it during lyophilisation, as the level of specific IgG in lyophilised formulations with trehalose are comparable to that of aluminium hydroxide Der p not lyophilised (Fig. 5, bottom panel). The same level of protection improvement is obtained for the examined concentrations of trehalose.

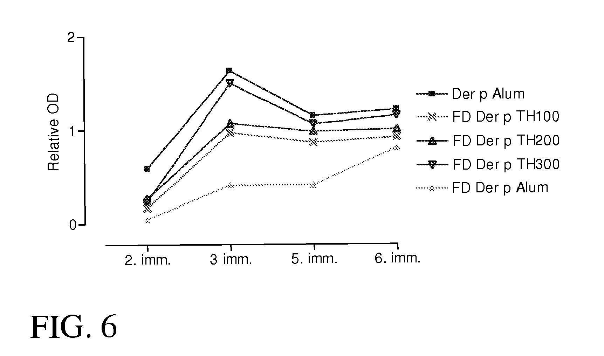

Fig. 6 illustrates the temporal development of the Der p 1 specific IgG during the course of the immunisation program. As mentioned before the onset of the immune response for the lyophilised aluminium hydroxide Der p formulation is much delayed compared to that of aluminium hydroxide Der p not subjected to lyophilisation. Addition of trehalose before lyophilisation approximates the development of the IgG response to the non-lyophilised formulation, with 300 mM trehalose as the preferable formulation.

Conclusion

Microscopy and Rocket Immunoelectrophoresis (RIE)

Analysis of aluminium hydroxide Phl p and aluminium hydroxide Der p show:

1. Trehalose does not seem to influence the gel particles when the samples are stored at 5°C.
2. The presence of trehalose during lyophilisation prevents the formation of crystals already at addition of 50mM trehalose.
3. Phase contrast images show that the gel particles of lyophilised and re-dissolved formulations maintain a similar structure as reference formulations not subjected to lyophilisation at trehalose concentrations above 150mM.

RIE of the supernatant of aluminium hydroxide Phl p samples show that the presence of trehalose induces desorption of Phl p and Phl p 6. However, microscopy of FITC labelled aluminium hydroxide Phl p showed that Phl p is associated to the gel particles.

RIE analysis of aluminium hydroxide Der p samples showed that there was no detectable desorption of Der p, Der p 1 and Der p 2.

IgE inhibition experiments

1. Trehalose protects the Der p Alutard vaccine formulation during lyophilization and the reconstituted vaccine is comparable to a reference preparation not subjected to lyophilisation with regard to potency and composition.
2. Trehalose does not elute proteins from the vaccine formulation.
3. Aluminium hydroxide vaccines lyophilized without trehalose in the mixture lose 85% of the potency when reconstituted in buffer

Mouse immunisations

1. For both aluminium hydroxide Phl p and Der p formulations it is shown that trehalose itself does not inhibit the immune response.

2. The onset of the immune response of lyophilised aluminium hydroxide Der p formulations is much delayed compared to that of aluminium hydroxide Der p not subjected to lyophilisation. Addition of trehalose before lyophilisation approximates the development of the IgG response to a non-lyophilised formulation, with 300 mM trehalose as the preferable formulation.

References

Axelsen N H, Kroll J and Weeke B; 1973; A Manual of Quantitative Immunoelectrophoresis-Methods and Applications; Scandinavian Journal of Immunology; vol. 2; Supplement no. 1

EMBODIMENTS

Item 1. A solid vaccine formulation adapted for mucosal administration and comprising at least one antigen as active substance, wherein the formulation comprises a lyophilisate of a suspension comprising an oxygen-containing metal salt, the antigen(s) and at least one excipient selected from (i) saccharides, (ii) sugar alcohols and (iii) amino acids or pharmaceutically acceptable salts thereof.

Item 2. The formulation according to item 1 wherein the suspension comprises a saccharide selected from the group consisting of dextrose, sucrose, lactose, trehalose, cellobiose, raffinose, isomaltose and cyclodextrins.

Item 3. The formulation according to item 2 wherein the suspension comprises a saccharide is selected from the group consisting of trehalose and sucrose.

Item 4. The formulation according to any of items 1-3 wherein the suspension comprises a sugar alcohol.

Item 5. The formulation according to item 4 wherein the suspension comprises a sugar alcohol selected from the group consisting of mannitol, sorbitol and allitol.

Item 6. The formulation according to any of items 1-5 wherein the suspension comprises an amino acid selected from glycine, alanine, glutamine, proline, serine, arginine, lysine and histidine and pharmaceutically acceptable salts thereof.

Item 7. The formulation according to any of items 1-6 wherein the suspension comprises an additional excipient which is a polymer, preferably dextran.

Item 8. The formulation according to any of the preceding items wherein the suspension comprises a mixture of:

(a) sucrose and mannitol,
(b) sucrose, glycine and dextran,
(c) trehalose and mannitol,
(d) sucrose, mannitol and dextran,
(e) trehalose, glycine and dextran,
(f) trehalose, mannitol and dextran,
(g) trehalose and glycine,
(h) sucrose and glycine, or
(i) glycine and mannitol.

Item 9. The formulation according to any of the preceding items wherein the antigen is an allergen.

Item 10. The formulation according to any of the preceding items wherein the oxygen-containing metal salt is aluminum hydroxide.

Item 11. The formulation according to any of the preceding items wherein the formulation is a tablet.

Item 12. The formulation according to item 11, wherein the tablet is a non-compressed tablet.

Item 13. The formulation according to item 12, wherein the non-compressed tablet is a fast-dissolving tablet.

Item 14. The formulation according to item 11, wherein the tablet is a compressed tablet.

Item 15. The formulation according to any of items 11-14, wherein the tablet is for sublingual administration.

Item 16. The formulation according to any of items 1-10, wherein the formulation is a powder.

Item 17. The formulation according to any of items 1-10, wherein the formulation is in the form of particles.

Item 18. The formulation according to any of items 1-10, wherein the formulation is a granulate.

Item 19. The formulation according to any of items 1-10 wherein the formulation is a capsule comprising the lyophilisate.

Item 20. Use of a lyophilisate of a suspension comprising an oxygen-containing metal salt, at least one antigen

and at least one excipient selected from (i) saccharides, (ii) sugar alcohols and (iii) amino acids or pharmaceutically acceptable salts thereof, for the preparation of a solid vaccine formulation for mucosal administration.

Item 21. The use according to item 20 wherein the suspension comprises a saccharide selected from the group consisting of dextrose, sucrose, lactose, trehalose, cellobiose, raffinose, isomaltose and cyclodextrins,

Item 22. The use according to item 21 wherein the saccharide is selected from the group consisting of trehalose and sucrose.

Item 23. The use according to any of items 20-22 wherein the suspension comprises a sugar alcohol.

Item 24. The use according to item 23 wherein the sugar alcohol is selected from the group consisting of mannitol, sorbitol and allitol.

Item 25. The use according to any of items 20-24 wherein the suspension comprises an amino acid selected from glycine, alanine, glutamine, proline, serine, arginine, lysine and histidine or a pharmaceutically acceptable salts thereof.

Item 26. The use according to any of items 20-25 wherein the suspension comprises an additional excipient which is a polymer, preferably dextran.

Item 27. The use according to any of the preceding items 20-26 wherein the suspension comprises a mixture of:

(a) sucrose and mannitol,
(b) sucrose, glycine and dextran,
(c) trehalose and mannitol,
(d) sucrose, mannitol and dextran,
(e) trehalose, mannitol and dextran,
(f) trehalose, glycine and dextran,
(g) trehalose and glycine,
(h) sucrose and glycine, or
(i) mannitol and glycine.

Item 28. The use according to any of the preceding items 20-27 wherein the antigen is an allergen.

Item 29. The use according to any of the preceding items 20-28 wherein the oxygen-containing metal salt is aluminium hydroxide.

Item 30. The use according to any of items 20-29 wherein the formulation is a tablet.

Item 31. The use according to item 30, wherein the tablet is a non-compressed tablet.

Item 32. The use according to item 31, wherein the non-compressed tablet is a fast-dissolving tablet.

Item 33. The use according to item 30, wherein the tablet is a compressed tablet.

Item 34. The use according to any of items 30-33 wherein the tablet is a tablet for sublingual administration.

Item 35. The use according to any of items 20-29, wherein the formulation is a powder.

Item 36. The use according to any of items 20-29, wherein the formulation is in the form of particles.

Item 37. The use according to any of items 20-29, wherein the formulation is a granulate.

Item 38. The use according to any of items 20-29 wherein the formulation is a capsule comprising the lyophilisate.

Item 39. A method for vaccination of a subject comprising mucosal administration of an effective amount of a solid formulation according to any of items 1-19.

Item 40. A method for treatment of allergy or alleviating symptoms of allergy comprising mucosal administration of an effective amount of a solid formulation according to items 1-19 comprising at least one allergen.

Item 41. The method according to any of items 39 or 40 wherein the administration is via the oromucosal mucosa.

Item 42. The method according to item 41 wherein the administration is via the sublingual mucosa.

Item 43. The method according to any of items 39-40 wherein the administration is oral and via the gastrointestinal mucosal membrane.

**Claims**

1.  A solid vaccine formulation adapted for mucosal administration and comprising at least one antigen as active substance, wherein the formulation comprises a lyophilisate of a suspension comprising an oxygen-containing metal salt, the antigen(s) and at least one excipient selected from (i) saccharides, (ii) sugar alcohols and (iii) amino acids or pharmaceutically acceptable salts thereof.

2.  The formulation according to claim 1 wherein the suspension comprises a saccharide selected from the group consisting of dextrose, sucrose, lactose, trehalose, cellobiose, raffinose, isomaltose and cyclodextrins.

3. The formulation according to claim 2 wherein the suspension comprises a saccharide is selected from the group consisting of trehalose and sucrose.

4. The formulation according to any of claims 1-3 wherein the suspension comprises an additional excipient which is a polymer, preferably dextran.

5. The formulation according to any of the preceding claims wherein the suspension comprises a mixture of:

   (a) sucrose and mannitol,
   (b) sucrose, glycine and dextran,
   (c) trehalose and mannitol,
   (d) sucrose, mannitol and dextran,
   (e) trehalose, glycine and dextran,
   (f) trehalose, mannitol and dextran,
   (g) trehalose and glycine,
   (h) sucrose and glycine, or
   (i) glycine and mannitol.

6. The formulation according to any of the preceding claims wherein the antigen is an allergen.

7. The formulation according to any of the preceding claims wherein the oxygen-containing metal salt is aluminum hydroxide.

8. Use of a lyophilisate of a suspension comprising an oxygen-containing metal salt, at least one antigen and at least one excipient selected from (i) saccharides, (ii) sugar alcohols and (iii) amino acids or pharmaceutically acceptable salts thereof, for the preparation of a solid vaccine formulation for mucosal administration.

9. The use according to claim 8 wherein the suspension comprises a saccharide selected from the group consisting of dextrose, sucrose, lactose, trehalose, cellobiose, raffinose, isomaltose and cyclodextrins,

10. The use according to claim 9 wherein the saccharide is selected from the group consisting of trehalose and sucrose.

11. The use according to any of claims 8-10 wherein the suspension comprises an additional excipient which is a polymer, preferably dextran.

12. The use according to any of the preceding claims 8-11 wherein the suspension comprises a mixture of:

   (a) sucrose and mannitol,
   (b) sucrose, glycine and dextran,
   (c) trehalose and mannitol,
   (d) sucrose, mannitol and dextran,
   (e) trehalose, mannitol and dextran,
   (f) trehalose, glycine and dextran,
   (g) trehalose and glycine,
   (h) sucrose and glycine, or
   (i) mannitol and glycine.

13. The use according to any of the preceding claims 8-12 wherein the antigen is an allergen.

14. The use according to any of the preceding claims 8-13 wherein the oxygen-containing metal salt is aluminium hydroxide.

Inhibition of the binding of IgE to biotinylated
Der p by various inhibitors.

FIG. 1

**Inhibition of the binding of IgE to biotinylated
Der p by various inhibitors**

Legend:
- ⊠ Der p IMP 7331_23,240205
- ⊙ Dp_Alu-TH_Lyo_240205
- ▽ Dp_Alu+TH_Lyo_240205
- ◆ Dp_Alu-TH_LyoSup_240205
- ⊙ Dp_Alu+TH_LyoSup_240205

## FIG. 2

## FIG. 3

FIG. 4

FIG. 5

FIG. 6

**Europäisches Patentamt**
**European Patent Office**
**Office européen des brevets**

## EUROPEAN SEARCH REPORT

Application Number

EP 11 15 5971

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 02/101412 A2 (POWDERJECT VACCINES INC [US]; POWDERJECT RES LTD [GB]) 19 December 2002 (2002-12-19) * page 5, lines 1-15 * * page 67; table 2.146 * * claims 1-13 * * page 19, lines 21-27 * | 1-14 | INV. A61K39/35 A61K9/00 |
| X | US 2003/202978 A1 (MAA YUH-FUN ET AL) 30 October 2003 (2003-10-30) * claims 1-13 * * page 26, right-hand column, paragraph 2 - page 27, left-hand column * * page 7, left-hand column, paragraph 2 * | 1-14 | |
| X,D | GRIBBON E M ET AL: "STABILISATION OF VACCINES USING TREHALOSE (Q-T4) TECHNOLOGY", DEVELOPMENTS IN BIOLOGICAL STANDARDIZATION, KARGER, BASEL, CH, vol. 87, 1996, pages 193-199, XP000867388, ISSN: 0301-5149 * page 194, paragraphs 4,6 * * page 197, paragraphs 3,4 * | 1-14 | |
| X,D | MAA YUH-FUN ET AL: "Optimization of an alum-adsorbed vaccine powder formulation for epidermal powder immunization.", PHARMACEUTICAL RESEARCH (DORDRECHT), vol. 20, no. 7, July 2003 (2003-07), pages 969-977, XP002364354, ISSN: 0724-8741 * page 971, right-hand column, paragraph 2 * * page 975, right-hand column, paragraph 2-3 * | 1-14 | |

TECHNICAL FIELDS SEARCHED (IPC)

A61K

-/--

The present search report has been drawn up for all claims

| Place of search | Date of completion of the search | Examiner |
|---|---|---|
| Munich | 12 April 2011 | Young, Astrid |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt

European Patent Office

Office européen des brevets

**EUROPEAN SEARCH REPORT**

Application Number

EP 11 15 5971

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 95/33488 A (QUADRANT HOLDINGS CAMBRIDGE LIMITED) 14 December 1995 (1995-12-14) * page 12, last paragraph - page 14, last paragraph * * claims 1-5,12 * ----- | 1-14 | |
| Y,D | WO 00/45847 A (ALK ABELLO A S [DK]; IPSEN HANS HENRIK [DK]; ULDAL RAHBEK JANNE [DK]) 10 August 2000 (2000-08-10) * claims 1-69 * * page 35, lines 6-27 * ----- | 1-14 | |

TECHNICAL FIELDS SEARCHED (IPC)

The present search report has been drawn up for all claims

| Place of search | Date of completion of the search | Examiner |
|---|---|---|
| Munich | 12 April 2011 | Young, Astrid |

EPO FORM 1503 03.82 (P04C01)

## ANNEX TO THE EUROPEAN SEARCH REPORT
## ON EUROPEAN PATENT APPLICATION NO.

EP 11 15 5971

This annex lists the patent family members relating to the patent documents cited in the above-mentioned European search report.
The members are as contained in the European Patent Office EDP file on
The European Patent Office is in no way liable for these particulars which are merely given for the purpose of information.

12-04-2011

| Patent document cited in search report | | Publication date | Patent family member(s) | | Publication date |
|---|---|---|---|---|---|
| WO 02101412 | A2 | 19-12-2002 | AU | 2002302814 A1 | 23-12-2002 |
| | | | CA | 2449593 A1 | 19-12-2002 |
| | | | EP | 1399131 A2 | 24-03-2004 |
| | | | JP | 2004532277 T | 21-10-2004 |
| US 2003202978 | A1 | 30-10-2003 | US | 2008038356 A1 | 14-02-2008 |
| WO 9533488 | A | 14-12-1995 | AT | 235919 T | 15-04-2003 |
| | | | CN | 1156967 A | 13-08-1997 |
| | | | DE | 69530196 D1 | 08-05-2003 |
| | | | DE | 69530196 T2 | 20-11-2003 |
| | | | DK | 762897 T3 | 21-07-2003 |
| | | | EP | 0762897 A1 | 19-03-1997 |
| | | | ES | 2194911 T3 | 01-12-2003 |
| | | | JP | 4142095 B2 | 27-08-2008 |
| | | | JP | 10500990 T | 27-01-1998 |
| | | | JP | 2008179636 A | 07-08-2008 |
| | | | PT | 762897 E | 31-07-2003 |
| | | | US | 2001055583 A1 | 27-12-2001 |
| WO 0045847 | A | 10-08-2000 | AU | 771552 B2 | 25-03-2004 |
| | | | AU | 2279700 A | 25-08-2000 |
| | | | CA | 2361377 A1 | 10-08-2000 |
| | | | CN | 1338947 A | 06-03-2002 |
| | | | EP | 1146906 A1 | 24-10-2001 |
| | | | JP | 2003517452 T | 27-05-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4578270 A
- US 5902565 A
- EP 0130619 B1
- WO 02101412 A
- WO 0193829 A
- WO 9533488 A
- WO 04047794 A
- WO 0045847 A
- EP 1516615 A
- US 5591433 A
- WO 9947680 A
- WO 0240676 A
- WO 03096869 A
- US 20030202978 A
- US 20050266021 A
- US 5648093 A
- WO 0051568 A
- WO 0213858 A
- WO 9921579 A
- WO 0044351 A
- US 4371516 A
- EP 278877 A
- WO 2004047794 A
- WO 2004075875 A

### Non-patent literature cited in the description

- **GRIBBON et al.** Dev Biol Stand. Basel. Karger, 1996, vol. 87, 193-199
- **MAA et al.** *Journal of Pharmaceutical Sciences,* February 2003, vol. 92 (2
- **MAA et al.** *Pharmaceutical Research,* July 2003, vol. 20 (7
- The Handbook of Chemistry and Physics. 1975
- **AXELSEN N H ; KROLL J ; WEEKE B.** A Manual of Quantitative Immunoelectrophoresis-Methods and Applications; Scandinavian Journal of Immunology. 1973, vol. 2